# EUROPEAN PATENT APPLICATION

(11) **EP 1 209 469 A1**
(43) Date of publication of application: **29.05.2002**
(21) Application number: 01127384.4
(22) Date of filing: 22.11.2001
(51) Int. Cl.: G01N 33/58, A61K 51/12, A61K 49/00

(54) **Production and use of a targeted diagnostic system**

(30) Priority: 22.11.2000 US 252666 P
(71) Applicant: Vectron Therapeutics AG, 35037 Marburg (DE)
(72) Inventor: Brüsselbach, Sabine, 35037 Marburg (DE); Fahr, Alfred, 35091 Cölbe (DE); Graser, Andreas, 35041 Marburg (DE)
(74) Representative: Bösl, Raphael, Dr. rer. nat., Dipl.-Chem.

(57) **Abstract**

The present invention concerns a targeted diagnostic system comprising a negatively charged carrier, a targeting moiety and a diagnostic agent and methods of producing and using the diagnostic system.

Sequence Listing

<110>  vectron therapeutics AG

<120>  Production and Use of a Targeted Diagnostic System

<130>  V34890EP

<150>  US 60252666
<151>  11/22/2000

<160>  3

<170>  WORD6.0, PC-DOS/MS-DOS

<210>  1
<211>  3
<212>  PRT
<213>  Homo sapiens

<400>  1

Arg Gly Asp

<210>  1
<211>  9
<212>  PRT
<213>  Artificial sequence

<220>
<221>  peptide used as targeting moiety

<400>  1

Cys Asp Cys Arg Gly Asp Cys Phe Cys  9

<210>  1
<211>  10
<212>  PRT
<213>  Artificial sequence

<220>
<221>  peptide used as targeting moiety

<400>  1

Arg Cys Asp Cys Arg Gly Asp Cys Phe Cys  10

## Description

### Field of the Invention

The present invention concerns a targeted diagnostic system comprising a negatively charged carrier, a targeting moiety and a diagnostic agent and methods of producing and using the diagnostic system. The targeted diagnostic system of the present inventions allows the specific delivery of diagnostic agents to only certain tissues and/or cell types and provides prolonged persistence of the diagnostic agent in the respective tissues and cell types. The targeted diagnostic system of the present invention is particularly suitable for diagnosis in tumor diseases, wherein a preferred target is the tumor endothelium.

### Description of the Related Art

Exact diagnosis of a disease is in general the prerequisite for an efficient and successful treatment of any disease. Such a diagnosis will in many cases not only require a qualitative result on whether, for example, a certain marker is present but the treatment will also depend on the spatial distribution of the marker within a patient. In particular in cancer diseases the identification of distant or near metastasis or the accurate determination of the perimeter of a tumor is a necessary precondition for the determination of the appropriate treatment regime like, for example, surgery, chemotherapy, radiotherapy and/or cryotherapy. To that end it has been suggested to selectively stain diseased tissue areas. Although, it has been difficult to target diagnostic agents to specific tissues or cell populations for extended periods of time.

This applies in particular to the specificity and efficiency of tumor cell targeting, indicating that the design of more efficacious and site-selective diagnostics is urgently required. In respect of diagnostic targeting of tumor cells a serious problem is the poor accessibility of many tumor cells to any kind of diagnostics, as a consequence of the defective tumor vasculature and the high interstitial pressure (Jain, R.K. (1998) *J. Controlled Release* **53**: 49-67). Liposomes have been employed as carriers for iodine compounds, for instance, and have been shown to yield extended residence times in the target areas, e.g. neoplastic tissue (Henze et al. (1989) Comput. Med. Imaging Graph 13, 455-462). More recently it has been suggested to use cationic liposome complexes to target diagnostics to tumor endothelial cells (Thurston et al. (1998) *J. Clin. Invest.* **101**: 1401-1413), i.e. endothelial cells forming vessels within a tumor, as a means to selectively stain diseased tissue areas. Such an approach potentially has several advantages one being that tumor blood vessels are more readily accessible than the actual tumor cell compartment, a second being that endothelial cells (ECs) are unknown to acquire resistance to treatment (Boehm, T., et al. (1997) *Nature* **390**: 404-7) and are, therefore, also less likely to become refractive to, for example, diagnostic staining and a third being that tumor endothelium represents a target that is largely independent of tumor type (Arap, W., et al. (1998) *Curr. Opin. Oncol.* **10**: 560-5).

However, all systems of particulate nature suffer from the fact that they are readily recognized by cells of the reticuloendothelial system, particularly macrophages in liver and spleen, but also by resident macrophages in the lung and other tissues. This leads to a high background signal (low signal-to-noise ratio) of the diagnostic agent, poor resolution and, correspondingly, difficult differential diagnosis. Ideally, a targeted diagnostic system should be safe, non-toxic, non-immunogenic and cell-type specific, it should mark and/or transduce the target cells with a high efficiency, and - with respect to clinical applicability - it should offer the possibility of large-scale production, stability of the product and ease of handling. To date, no diagnostic system is available that would accomplish high target selectivity and low systemic distribution, particularly in organs rich in reticuloendothelial cells.

In an effort to increase the circulation time of liposomes the effect of poly(ethylene glycol) (PEG) has been examined in mice by employing amphipathic PEGs (phosphatidylethanolamine (PE) derivatives of PEG) with average molecular weights of 1000, 2000, 5000 and 12,000 (Maruyama, K.T et al. (1992) *Biochem. Biophys. Acta* **1128:** 44-9). The activity of dioleoyl phosphatidylethanolamine-PEG (DOPE-PEG) in prolonging the circulation time of liposomes was proportional to the molecular weight of PEG, i.e., 12000 = 5000 greater than 2000 greater than 1000. The structure-function relationship of PEG-derivatized phosphatidylethanolamine (PEG-PE) has been examined by measurement of blood lifetime and tissue distribution in both mice and rats has also been reviewed in (Woodle, M.C.and Lasic. D.D.(1992) *Biochim. Biophys. Acta* **1113**: 171-99 and Woodle, M.C. and Matthay K.K. (1992) *Biochim. Biophs. Acta* **1105**: 193-2000). The effect of different molar content and chain length of a PEG-Chol derivatives has been studied in cultured macrophage cell line J774 (Vertut-Doi, A.H. Ishiwata H. et al. (1996) *Biochim. Biophys. Acta* **1278:** 19-28). In this study it was found that liposomes containing PEG-Chol decreased the binding and endocytosis of the liposomes.

In other work lipid-conjugates of two amphipatic polymers, poly(2-methyl-2-oxazoline) (PMOZ) and poly(2-ethyl-2-oxazoline) (PEOZ) were synthesized by linking glutarate esters of the polymers to distearoylphosphatidylethanolamine (DSPE) or alternatively by termination of the polymerization process with DSPE (Woodle, M.C. Engbers C.M. et al. (1994) *Bioconjug. Chem.* 5: 493-6). When injected into rats both polymers PEOZ and PMOZ were found to convey long circulation and low hepatosplenic uptake to liposomes. Other lipid derivatives of polyethyleneglycol (PEG) have been synthesized and tested for the ability to allow liposomes to evade uptake by the reticuloendothelial system (RES) and to prolong the circulation time of liposomes in mice (Yuda, T.K. et al. (1996) *Biol. Pharm. Bull.* **19:** 1347-51). Novel conjugates for inclusion in liposomal formulations, containing distearoylphosphatidylethanolamine (DSPE) as a lipid anchor, heterobifunctional polyethylene glycol (PEG) with a molecular weight of 2000 as a linking moiety, and a biological cell adhesive ligand [YIGSR peptide or Sialyl Lewis(X) oligosaccharide (SLX)], were synthesized (Zalipsky, S.N. et al. (1997) *Bioconjug. Chem.* **8**: 111-8).

### Brief Summary of the Invention

A diagnostic system comprising: (a) at least one negatively charged carrier, (b) at least one targeting moiety and (c) at least one diagnostic agent.

In one embodiment of the diagnostic system of the present invention said negatively charged carrier is selected from the group consisting of, phosphatidylserine, phosphatidylglycerol, phosphatidylacid, phosphatidylinositol, and cholesterolglutarate.

The diagnostic system of the present invention, wherein said targeting moiety is selected from the group consisting of a peptide, a protein, an antibody, an antibody fragment, a single-chain antibody, a diabody or a small molecule.

The diagnostic system of the present invention, wherein said diagnostic agent is selected from the group consisting of an electron dense substance, a paramagnetic substance, a superparamagnetic substance, a radioactive substance, a fluorescent substance, a luminescent or otherwise light-emitting substance, a genetic construct that codes for a fluorescent protein and a genetic construct that codes for a protein detectable by staining procedures.

The diagnostic system of the present invention, further comprising at least one uncharged carrier.

In one embodiment of the diagnostic system of the present invention said uncharged carrier is selected from the group consisting of cholesterol, phosphatidylcholine and phosphatidylethanolamine.

The diagnostic system of the present invention, wherein said uncharged carrier is selected from the group consisting of cholesterol, phosphatidylcholine, and phosphatidylethanolamine.

The diagnostic system of the present invention, further comprising at least one amphiphatic polymer.

In a preferred embodiment of the diagnostic system of the present invention said amphiphatic polymer is selected from the group of polyethylene glycol (PEG), poly(2-methyl-2-oxazoline) (PMOZ), and poly(2-ethyl-2-oxazoline) PEOZ.

In one embodiment of the diagnostic system of the present invention said amphiphatic polymer is attached to a lipid.

In one embodiment of the diagnostic system of the present invention said lipid is selected from alpha-methoxy-omega-(1,2-dioctadecenoyloxy glyceryl) (DO), alpha- methoxy-omega-(1,2-ditetradecenoyloxy glyceryl) (DT), distearoylphosphatidyl (DSPE), and alpha-(dipalmitoylphosphatidyl) (DPP).

In a preferred embodiment of the diagnostic system of the present invention said amphiphatic polymer has a molecular weight between 1.000 and 5.000 Da.

The diagnostic system of the present invention, wherein the negative carrier of the diagnostic system forms at least a part of a lamellar structure.

In one embodiment of the diagnostic system of the present invention said lamellar structure is a unilamellar, bilamellar, oligolamellar or polylamellar structure.

In one embodiment of the diagnostic system of the present invention said lamellar structure is a liposome.

In one embodiment of the diagnostic system of the present invention said lamellar structure is an artificial viral envelope.

In one embodiment of the diagnostic system of the present invention said lamellar structure comprises a payload compartment.

In a preferred embodiment of the diagnostic system of the present invention said diagnostic agent is encapsulated within said payload compartment.

In one embodiment of the diagnostic system of the present invention said at least one targeting moiety is covalently attached to at least one of said negatively charged carriers or uncharged carriers.

A method for producing the diagnostic system of the present invention, wherein at least one lamella is formed with at least one negatively charged carrier and at least one diagnostic agent in solution.

The method for producing the diagnostic system of the present invention, wherein at least one targeting moiety is covalently attached to at least one of said negatively charged carrier.

In one embodiment of the method for producing the diagnostic system of the present invention said at least one targeting moiety is covalently attached after formation of at least one lamella.

The method for producing the diagnostic system of the present invention, wherein at least one targeting moiety is mixed with said at least one negatively charged carrier.

A method for producing of the diagnostic system of the present invention, wherein at least one lamella is loaded with at least one diagnostic agent upon formation of the lamella.

A method of using the diagnostic system of the present invention for the diagnosis of a disease.

In one embodiment of the method of using the diagnostic system of the present invention the disease is a tumor disease.

A method of using the diagnostic system of the present invention for diagnosis of cells and tissues.

A method of using the diagnostic system of the present invention for the diagnosis of microbiological organisms in vivo.

A diagnostic kit comprising at least one negatively charged carrier and at least one diagnostic agent.

In one embodiment the diagnostic kit of the present invention further comprises at least one targeting moiety.

### Brief Description of the Drawings

- ***Figure 1:***: Condensation of DNA by polyethylenimine determined by dye exclusion. Six individual measurements with different DNA plasmid concentrations are shown to illustrate the high reproducibility of the method.
- ***Figure 2:***: Size determination of RGD-AVEs, PEI-DNA and RGD-AVPs by photon correlation spectroscopy. The mean values for the size distributions estimated by z-average calculation are indicated.
- ***Figure 3:***: Cryogenic transmission electron microscopy (Cryo-TEM) of RGD-AVPs complexed with PEI/plasmid-DNA. The pictures shows encapsulation of condensed DNA in a lipid layer within discrete particles. The diameter of the larger liposomes shown are approx. 140 nm.
- ***Figure 4:***: Toxicity of AVPs for HUVECs compared to Lipofectamin (LA; Gibco BRL) and Superfect (SF; Quiagen, Hilden Germany). The analyses were performed at 6 h and 24 h after transfection. Identification of apoptotic cells by staining with Hoechst 33258. Control, untransfected cells.
- ***Figure 5:***: Toxicity of AVPs for HUVECs compared to Lipofectamin (LA; Gibco BRL) and Superfect (SF; Quiagen, Hilden Germany). The analyses were performed at 6 h and 24 h after transfection. Determination of metabolic activity by WST-assay. The following amounts of DNA and transfection reagents were used (per 3 cm well): AVP, 5 µg DNA + 20 µg RGD-AVE; LA, 2 µg DNA + 20 µg lipid; SF, 2 µg DNA + 30 µg of the transfection reagent.
- ***Figure 6:***: Binding of AVPs to HUVECs. HUVECs were exposed for 1 h to NBD-PE labeled AVPs with and without RGD ligand and analyzed by fluorescence microscopy either directly (1 h) or after another 2 h in normal culture medium (3 h).
- ***Figure 7:***: Transduction of HUVECs by RGD-AVPs carrying a CMV-H2BGFP plasmid. Cells were exposed for 1 h to the AVPs and fluorescence microscopy was performed after another 23 h in normal medium (left panel). The right panel shows the same cells stained with Hoechst 33258.
- ***Figure 8:***: FACS-analysis of HUVECs 24 h after transduction with AVPs (± RGD ligand) carrying a CMV-H2BGFP plasmid compared to untreated cells. Cells were exposed for 1 h to the AVPs and FACS analysis was performed after another 23 h in normal medium.
- ***Figure 9:***: Transduction of different cell types with AVPs (A) RGD-AVPs (B) carrying a CMV promoter-H2BGFP plasmid: summary of FACS analyses determining the fraction of GFP-positive cells. Cells were exposed for 1 h to the AVPs and FACS-analysis was performed after another 23 h in normal medium. Values were calculated relative to the transduction efficiency obtained with RGD-AVP transfected HUVECs (100%).
- ***Figure 10:***: FACS-analysis of αᵥβ₃ expression in HUVECs and different tumor cell lines after indirect immunostaining as described in Materials and Methods. Bold lines: specific staining (first antibody: αᵥβ₃-specific; second antibody: Cy3-labeled); thin line: unspecific staining (second antibody only); dotted line: unlabeled cells. The relative fraction of cells is plotted against the intensity of fluorescence.
- ***Figure 11:***: T1-relaxation times in three different cell lines after treatment with EPC-Gd liposomes (EPC Gd), free Gd (Gd), AVE-RGD liposomes (AVE-RGD) and AVE-RGD Gd liposomes (AVE-RGD Gd).

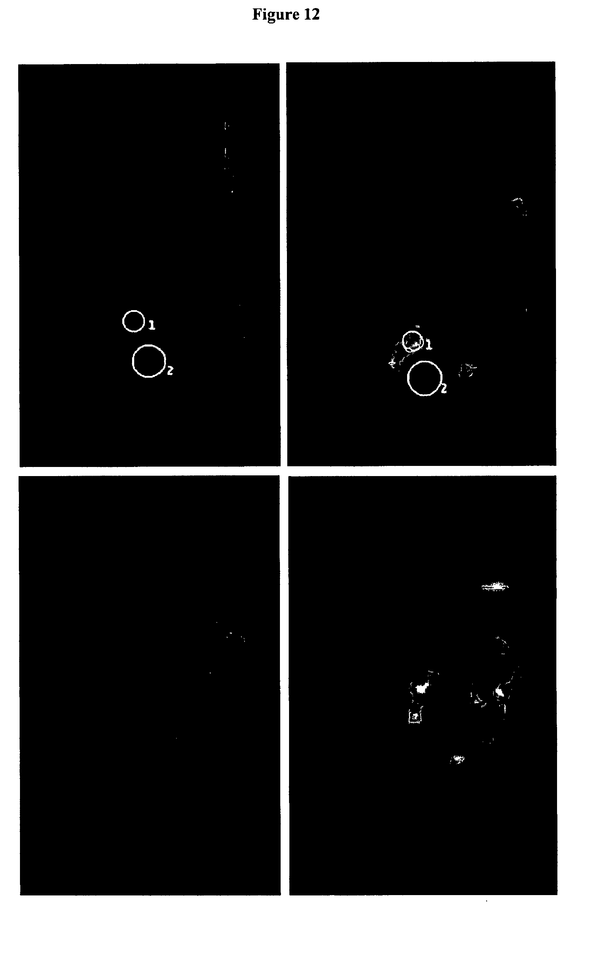

- ***Figure 12:***: Detection of a subcutaneous MEWO tumor in a mouse with AVE-PEG-RGD Gd-liposomes after intravenous injection of the liposomal formulation. The images on the left side were taken before the injection. The two images on the right side were taken 20 minutes after the injection. The upper and the lower images show two different sections of the same mouse. Both circle 1 and circle 2 in the upper images are located within the subcutaneous MEWO tumor. The subpopulation of tumor cells in circle 2 will also accumulate AVE-PEG-RGD Gd-liposomes after 1 h (Figure not shown). The white region in the lower right image lies within the MEWO tumor.

### Detailed Description of the Invention

Liposomes carrying iodine or antibodies directed against certain disease markers have been used for targeted diagnosis with the disadvantage of poor targeting and/or short persistence in the targeted tissue and/or cells. The targeted diagnostic system of the present invention comprising a negatively charged carrier, a targeting moiety and a diagnostic agent, for example, improves the persistence of diagnostic agent in the cell and/or tissue, has a low toxicity, a long shelf life and increases the specificity of the targeting of the diagnostic agent.

A rate-limiting step of diagnostic in vivo analysis is the residence time of the diagnostic agent at the site which is determined by binding affinity to the target, desorption from the target and rate of elimination (which is often limited by the rate of blood flow). This equilibrium can be favorably changed towards longer residence times and higher intensities when the diagnostic agent is (a) selectively carried to the target site and does not bind in appreciable amounts to any non-target areas; (b) binding affinity to the target cell population is high; and (c) the carrier is capable of intracellularly depositing the diagnostic agent, thus eliminating the wash-out as a rate-limiting factor.

One embodiment of the targeted diagnostic system of the present invention are artificial viral envelopes (AVEs) (US 5,252,348; US 5,766,625; US 5,753,258; EP 0 555 333 B1), that encapsulate at least one diagnostic agent. AVEs mimic the lipid composition of retroviruses. These natural lipids are anionic and, in contrast to their artificial cationic counterparts, interact only weakly with their biological environment and are, therefore, non-toxic. Due to the fact that they carry a negative surface charge (as a consequence of their lipid composition) they are ideally suited to attach targeting moieties to, or insert them stably into the lipid layer. In addition the artificial viral envelops (AVEs) are equipped with a targeting moiety directed at, for example, tumor endothelial cells or activated endothelial cells. In case of EC targeting, for example, a cyclic RGD peptide (Pasqualini, R., et al. (1997) *Nat. Biotechnol.* **15**: 542-6) can be used, that is thought to interact with αᵥβ₃ integrin.

Negatively charged carriers of the present invention are chemical compounds comprising at least one negatively charged region and at least one hydrophobic region, that can form lamellar structures, in particular unilamellar, bilamellar, oligolamellar or multilamellar lamellar structures. The terms "lamella" and "layer" are used synonymously in the present invention.

In a further embodiment the lamellar structure is a unilamellar, bilamellar, oligolamellar or multilamellar vesicles.

The lamellar structures of the present invention can also be formed by an even number of lamellas like, for example, 2, 4, 6, 8, 10, or 12 or an uneven number of lamellas like, for example, 1, 3, 5, 7, 9, or 11 lamellas wherein one site is facing a hydrophobic interior and the other site is facing the hydrophilic exterior of a suspension. This type of lamellas are, for example, suitable for the delivery of hydrophobic diagnostic agents.

Examples of such negatively charged carriers are anionic lipids, in particular but not limited to phosphatidylserine, phosphatidylglycerol, phosphatidylacid, phosphatidylinositol, or cholesterolglutarate.

In one embodiment the diagnostic system comprises a mixture of at least two different negatively charged carriers. Suitable negatively charged carriers that can be mixed are, for example, phosphatidylglycerol and phosphatidylserine.

The diagnostic system of the present invention can further comprise uncharged carriers that are incorporated into the lamellar structure like, for example, neutral lipids. Neutral lipids that can be incorporated into the lamellar structure of the present invention are, for example, but not limited to glycerophospholipids, in particular phosphatidylcholin, steroids, in particular chloesterol, glycerophosphonolipids, glycerophosphinolipids, sphingolipids, phosphatidylglycerol and/or phosphatidylethanolamine. A particularly, preferred, neutral lipid, which can form part of the lamellar structure is cholesterol.

The diagnostic system of the present invention can further comprise at least one amphiphatic polymer, which decreases the uptake of the diagnostic system by macrophages and, thus, increases the live span of the diagnostic system of the present invention in the circulation in *in vivo* applications.

In a preferred embodiment of the diagnostic system of the present invention said amphiphatic polymer is selected from the group of polyethylene glycol (PEG), poly(2-methyl-2-oxazoline) (PMOZ), and poly(2-ethyl-2-oxazoline) PEOZ. A particularly preferred amphiphatic polymer that can be used in the present invention is PEG.

The amphiphatic polymer can be attached to any component forming the diagnostic system as long as it allows presentation of the amphiphatic polymer on the outside of the diagnostic system. The linkage can be covalently or non-covalently, however, a covalent linkage between the amphiphatic polymer and the component of the diagnostic system that it is linked to is preferred. Particularly suitable for attachment of the amphiphatic polymer are negatively charged carriers or uncharged carriers. In a preferred embodiment of the diagnostic system of the present invention the amphiphatic polymer is attached to a lipid, preferably a anionic lipid or a neutral lipid. Suitable lipids are, for example, alpha-methoxy-omega-(1,2-dioctadecenoyloxy glyceryl) (DO), alpha- methoxy-omega-(1,2-ditetradecenoyloxy glyceryl) (DT), distearoylphosphatidyl (DSPE), and alpha-(dipalmitoylphosphatidyl) (DPP). Particularly preferred combinations of lipids and amphiphatic polymers are alpha-methoxy-omega-(1,2- dioctadecenoyloxy glyceryl)polyoxyethylene (DO-PEG) and alpha- methoxy-omega-(1,2-ditetradecenoyloxy glyceryl) polyoxyethylene (DT- PEG), in which PEG is directly linked to glycerol, and distearoylphosphatidyl-N-(methoxy polyoxyethylene succinyl)-ethanolamine (DSPE-PEG). The most preferred lipid for coupling of the amphiphatic polymer is DSPE.

In one embodiment the amphiphatic polymer can further comprise a group, which allows coupling of at least one targeting moiety of the present invention to the amphiphatic polymer, thereby combining the targeting function provided by the targeting moiety and the increase of circulation time provided by the amphiphatic polymer in one molecule. For example, PEG-derivatives with a functional group at the PEG terminal, which allow such further coupling reactions are distearoyl-phosphatidyl- N-(3-carboxypropionyl polyoxyethylene succinyl)ethanolamine (DSPE-PEG-COOH) and alpha-(dipalmitoylphosphatidyl)-omega-hydroxypolyoxyethylene (DPP-PEG-OH).

The molecular weight of the amphipatic polymer, in particular PEG, is in a preferred embodiment of the diagnostic system of the present invention between approx. 500 Da and approx. 50.000 Da, more preferably between approx. 750 Da and approx. 10.000 Da and even more preferably between approx. 1.000 Da and approx. 5.000 Da. If an amphiphatic polymer, in particular PEG, is included in the diagnostic system of the present invention targeting is most efficient, if the amphiphatic polymer is between approx. 2.000 Da and approx. 3.500 Da.

The amphiphatic polymer can be coupled to one of the components of the diagnostic system of the present invention prior or after addition of the individual components. However in a preferred embodiment the amphiphatic polymer is already coupled to one of the component of the diagnostic system when it is added. It is also possible to add lipid coupled amphiphatic polymers like, for example, PEG-lipids to preformed liposomes. Upon incubation with the liposomes the PEG-lipid will "slip" into the preformed liposome and be retained within the liposome.

In a preferred embodiment the amphiphatic polymer coupled to a component of the diagnostic system, in particular a lipid coupled amphiphatic polymer, constitutes between approx. 1 and approx. 30 mol% of the lipid components of the diagnostic system, more preferred between approx. 3 and approx. 20 mol%, even more preferred between approx. 5 and approx. 15 mol% and most preferred approx. 10 mol%.

The diagnostic system of the present invention can further comprise proteins that are incorporated into the lamellar structure like, for example, proteins with at least one membrane spanning domain.

Any compound that is incorporated into the lamellar structure can contain at least one group that allows coupling of targeting moieties. In particular the negatively charged and/or uncharged carriers of the present invention are compounds, which can contain at least one group that allows covalent or non-covalent coupling of targeting moieties like, for example, coupling via carboxylic acid amid bonds (reversible covalent coupling with, for example, N-glutarylphosphatidylethanolamine; Bogdanov, A. A. et al. (1988) *FEBS Lett.* **231:** 381 -384), biotin-avidin (non-covalent coupling; see, for example, Urdal, D. L., Hakomori, S. (1980), *J. Biol. Chem.* **255:** 10509-10515), coupling with hydrazide containing reagents (reversible covalent coupling; see, for example, Chua, M.-M. et al. (1984) *Biochim. Biophys. Acta* **800:** 291-300), coupling via disulfid bond(s), coupling via thioether bonds (irreversible covalent coupling; see, for example, Harokopakis E. et al. (1995) *J. Immunol Methods* **185:** 31-42; Martin, F. J. and Papahadjopoulos, D. (1982) *J. Biol. Chem.* **257:** 286-288; Hansen, C. B., et al. (1995) *Biochim. Biophys. Acta* **1239:** 133-144)

In a preferred embodiment only a fraction of the compounds incorporated into lamellar structure will contain at least one group that allows coupling of targeting moieties. In particular between about 1% to about 50 %, more preferred about 5% to about 20%, in particular about 10% of the compounds incorporated into a lamellar structure will contain at least one group that allows coupling of targeting moieties.

In one embodiment of the diagnostic system of the present invention the lamellar structure comprises at least one negatively charged carrier within the molar ratio of 100% to 5%. In a preferred embodiment the lamellar structure comprises at least one negatively charged carrier in a molar ratio of less than about 70%, in particular less than about 60%, preferably less than about 50%, more preferably less than about 40% and most preferably less than about 30%.

In a preferred embodiment of the diagnostic system of the present invention, wherein the lamellar structure comprises less than 100% of the negatively charged carrier, the remainder of the lamellar structure is composed of uncharged carriers, in particular of at lest one neutral lipid. While one or more negatively charged carrier in particular negatively charged lipids, can be combined with only one type of neutral carrier, in particular with neutral lipids, more preferably with cholesterol. It is preferred to combine one or more negatively charged carrier, in particular one or more negatively charged lipid, with two or more neutral carriers, in particular with neutral lipids, more preferably with a mixture of cholesterol and phosphatidylethanolamine.

It is preferred that the ratio of negatively charged carrier, in particular negatively charged lipid, to neutral carrier, in particular neutral lipid, within the lamellar structure is between 10-0.1 (negatively charged: neutral), preferably about 1-0.15, more preferably 0.8-0.2, more preferably about 0.5-0.25, most preferably about 0.4-0.3.

In particularly preferred embodiment of the diagnostic system of the present invention the molar ratios of the components of the lamellar structure phosphatidylserine, phosphatidylethanolamine, cholesterol and phosphoethanolamine-N-(Glutaryl) are 3:3:3:1.

Diagnostic agents that are comprised in the diagnostic system of the present invention are, for example, but not limited to electron dense molecules, paramagnetic, superparamagnetic or radioactive molecules, fluorescent agents, or nucleic acid constructs encoding fluorescent proteins or stainable peptides (less than 50 amino acids) or proteins (more than 50 amino acids). Examples are Europium complexes as a paramagnetic agent (Nomicos, C.D. (1998) **8(2)**: 313-318) or Iotrolan and Iopamidol as radioactive molecules ( Boni, L.T. (1991) Invest. Radiol., **26** Supl 1: 169-171). Particularly suitable are, for example, diagnostic agents that are detectable by medical imaging systems, e.g. x-ray, nuclear magnetic resonance and computer tomogram like, for example, Gadolinium-complexes. The skilled person knows a variety of diagnostic agents that could equally be employed in the present invention. Suitable fluorescent proteins are, for example, green-fluorescent protein (GFP) or its derivatives that emit fluorescent light with a different wave length and/or that have a shifted absorption maximum, i.e. that are exited at a different wave length.

The most preferred embodiment of the invention employs , paramagnetic, superparamagnetic or radioactive molecules, which can be visualized with non-invasive methods using medical imaging systems, e.g. x-ray, nuclear magnetic resonance and computer tomogram.

In some cases it might not be possible to detect the diagnostic agent directly in the transduced cells because, for example, the cell mass is very small. In these cases the diagnostic agent can comprise a nucleic acid encoding a secreted protein that can be detected in, for example, the blood of the patient. The detection of very small cell masses can be necessary, for example, after operation of prostate carcinoma or testicular carcinoma, wherein only a few cancer cells might be released into the body. Since the targeted diagnostic will preferably transduce the released prostate or testicular carcinoma cells a sustained increase in the secreted protein will indicate the presence of released cancer cells and will allow a more accurate decision on whether further treatment like, for example, chemo- or radiotherapy is necessary.

The targeting moiety can be directed against any substance that is present on the surface of a tissue and/or a cell (hereinafter target). For most diagnostic purposes it will be desirable to select a target that is preferentially present in tissues and/or cells that are diseased or in tissues and/or cells that are adjacent to diseased tissues and/or cells, thereby allowing selective targeting of the targeted diagnostic system of the present invention primarily to those tissues and/or cells, that are diseased or are indicative of a disease. Someone of skill in the art is aware of a variety of targets that are preferentially present in tissues and/or cells that are diseased or in tissues and/or cells that are adjacent to diseased tissues and/or cells. The targeting moiety that binds to this target can be, for example, a peptide , a protein as EGF (Fuwa T. et al. (1996) Bioch. Biophys. Res. Com., **227**: 666-671, an antibody like the 34 A monoclonal antibody or the monoclonal antibody 21B2 (Moribe K. et al (1999) Adv. Drug Del. Rev., **40**: 89-102), an antibody fragment ( Hudson, P. (1998) Current opinion in Biotechnology, **9**: 395-402, a single-chain antibody (Schier R. et al. (1999) J. of Immunological Methods, **231**: 249-260), a diabody or a small molecule like folate (Low, P.S. et al. (1994) J. Biol. Chem., **269**: 3198-3204).

In one embodiment of the diagnostic system of the present invention the small molecules are compounds that are not proteinaceous in nature and can bind to the surface of the targeted cell and/or tissue. In particular the small molecules can bind to targets, for example, receptors that are present on the surface of the cell and/or tissue. Examples of such small molecules are, but not limited to hormones and signaling molecules.

The targeting moiety can be coupled to any compound forming the lamellar structure, preferred is the coupling to uncharged or negatively charged carriers, for example, prior, during or after formation of the lamellar structure. Alternatively it can be coupled to a substance that is later incorporated into the lamellar structure like, for example, a lipid, in particular a neutral or negatively charged lipid or it can naturally be contained in or fused to a region that is incorporated into the lamellar structure like, for example, a hydrophobic membrane spanning region of a protein.

Several marker proteins have been identified, that are upregulated in tumor endothelium. Among these membrane-associated proteins are of particular interest, because they can be exploited for targeting the tumor vasculature. Examples are vascular endothelial growth factor receptor II (FLK-1, KDR) (Plate, K.H. *et al*.(1994) *Int. J. Cancer* **59**: 520-9), transforming growth factor binding protein CD105/endoglin (Burrows, F.J, *et al.* (1995) *Clin. Cancer Res.* **1**: 1623-34; Miller, D.W, *et al.* (1999) *Int. J. Cancer* **81:** 568-72), αᵥβ₃ integrin (Ruoslahti, E. (1999) *Adv. Cancer Res.* **76**: 1-20), transferrin receptor (Cotten, M. et al. (1994) Adv. Drug Del.. Rev., **14** : 113-136) and CD13/aminopeptidase N (Pasqualini, R., *et al.* (2000) *Cancer Res.* **60**: 722-7).

The diagnostic system of the present invention can further comprise substances that provide fusogenic properties, which facilitate fusion of the diagnostic system with the targeted cell membrane or endosomolytic properties that facilitate escape of the diagnostic system from the endosomes. A suitable compound providing endosomolytic properties are the cationic polymers like, for example, polyethyleneimine (PEI) (Boussif, O., *et al.* (1995) *Proc. Natl. Acad. Sci. USA* **92**: 7297-301), which on one hand condenses the DNA but also acts as an endosomolytic compound (Behr, J. (1997). *Chimia* ja **51:** 34-36. Preferably low molecular weight (LMW) PEI is used that has a molecular weight in the range from about 1.000 D to about 100.000 D, more preferably from about 2.000 D to about 50.000 D, more preferably about 5.000 to about 25.000 D. The inclusion of PEI, preferably LMW PEI, in the diagnostic system of the present invention is preferred, when the diagnostic agent is DNA. However, the endosomolytic properties of PEI are also desirable, if the diagnostic agent is, for example, a protein or an electron dense molecule.

In one embodiment the diagnostic system of the present invention can be used to diagnose the absence, increase, decrease or presence of certain cell types and/or tissues within a mammal, i.e. *in vivo.* The cell types can be, for example, tumor cells, microorganisms, infected cells, in particular virus infected cells or cells that show an aberration of metabolic functions.

Beside the determination of the absence, increase, decrease or presence of certain cell types and/or tissues the diagnostic read out of the diagnostic system can also be the increase or decrease of the amount of the diagnostic agent that can be detected in the target cells and/or tissues. Therefore, in another embodiment the diagnostic system of the present invention can be used to diagnose the increase of decrease of at least one diagnostic agent in a cell and/or tissue.

In one embodiment the diagnostic system of the present invention can be used to diagnose the absence of presence of certain cell types or tissues *in vitro.* This type of in vitro diagnosis can be used, for example, for the *in vitro* analysis of biopsies of a patient for tumor cells and/or tumor tissue or for analysis or tissue culture cells established from a patient to detect the presence or absence of specific tissue and/or cells.

The following examples merely serve to further illustrate the invention and in no way serve to limit the scope of the invention

### Examples

### 1. Construction and physico-chemical properties of AVEs

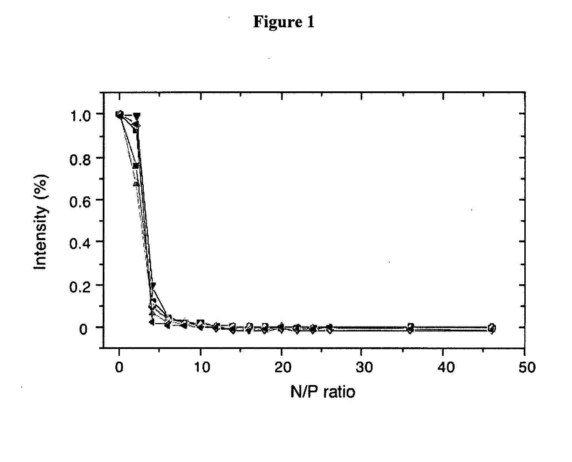

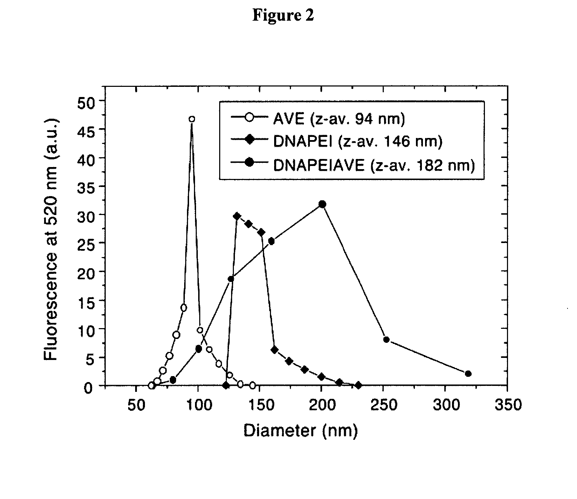

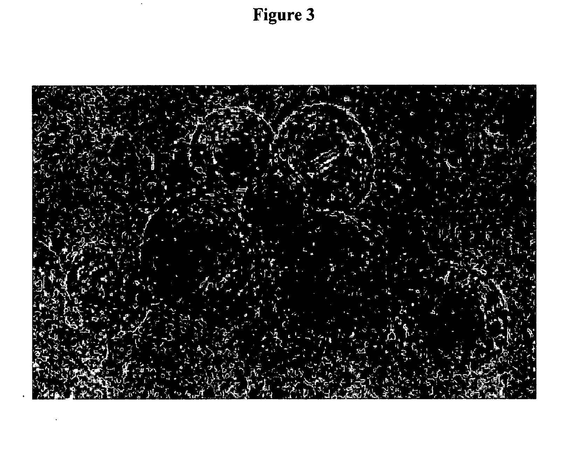

Unilamellar vesicles were prepared from dry lipid films of phosphatidylethanolamine, phosphatidylserine and cholesterol, which represent major constituents of the HIV envelope, and extruded using a LiposoFast™ extruder. The cyclic ligand with the RGD motif (Pasqualini, R., et al. (1997) *Nat. Biotechnol.* **15**: 542-6) has coupled to the liposomes via an anchor lipid containing a glutaric acid group. Coupling efficiency was ∼1 mol%, corresponding to ∼1000 peptide ligands per liposome, as judged by coupling a fluorescent derivative of the peptide to the liposomal surface. The average size of these RGD-AVEs was 94 nm according to photon correlation spectroscopy analysis (Fig. 1). The zeta potential was -45 mV. The AVEs could be stored for >2 months without loss of activity and did not exhibit any size increase. Plasmid DNA was condensed by mixing with small molecular mass non-linear polyethyleneimine (PEI) (Boussif, O., *et al.* (1995) *Proc. Natl. Acad. Sci. USA* **92:** 7297-301) (or protamine sulfate for comparison). DNA condensation was already achieved at a N/P-ratio of ∼8, as judged by dye exclusion (Fig. 2). At a N/P-ratio of 20.7, which was chosen for all subsequent experiments, DNA condensation was complete (Fig. 2). The mean size of this complex was 146 nm (Fig. 1). Packaging of this PEI/DNA plasmid complex into the RGD-AVEs gave rise to a moderate size increase and yielded particles with a mean size of 182 nm (Fig. 2). These fully assembled particles will subsequently be referred to as artificial viral particles (AVPs). The expected structure of the AVPs, i.e. the encapsulation of condensed DNA in a lipid layer within discrete particles, was confirmed by cryoelectron microscopy (Fig. 3). The AVPs, were stable for at least 350 h in solution as measured by photon correlation spectroscopy (data not shown).

### 2. Toxicity for endothelial cells

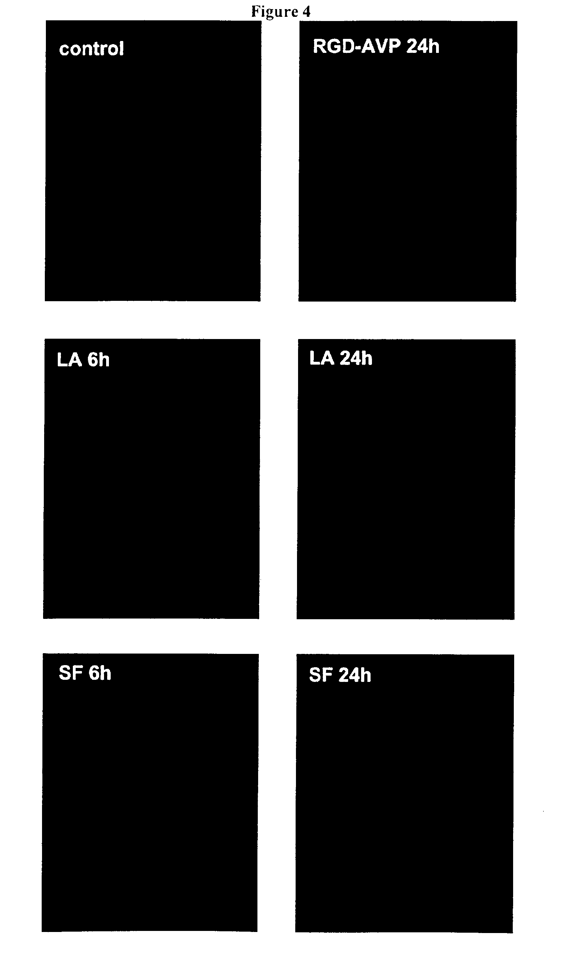

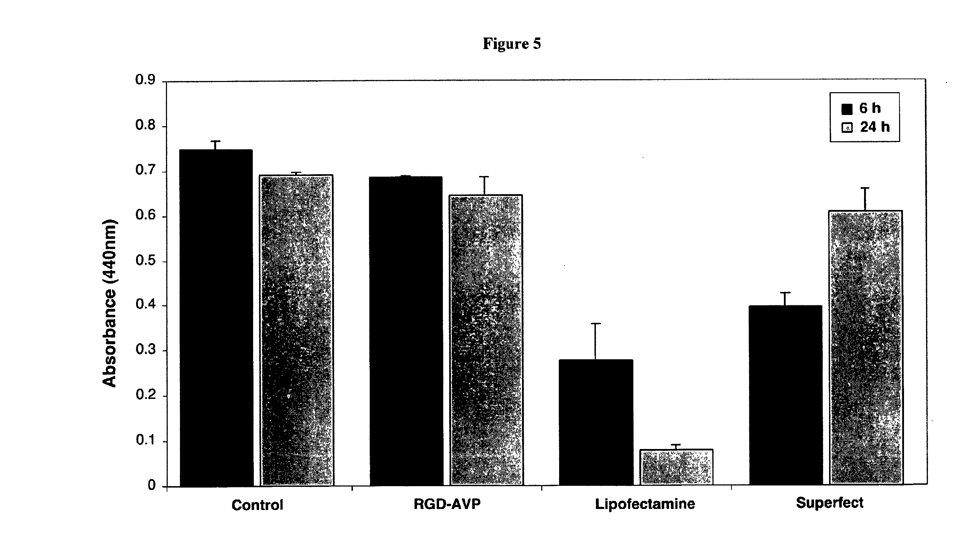

To assess potential toxicities the RGD-AVPs were incubated with cultures of HUVECs for 6 h and observed for another 18 h. No indication for RGD-AVP-induced cell death could be detected, as suggested by the absence of apoptotic cells identified by staining with Hoechst 33258 (Fig. 4). In addition, no influence on metabolic activity of the cells in the presence or absence of the RGD-AVPs was seen, as evidenced by WST assay (Fig. 5). In contrast, complexes of DNA with commercially available cationic lipids (Lipofectamine, Lipofectin) or polymers (Effectene, Superfect) killed the majority of the cells within the same period of time (Fig. 4 and 5 and data not shown).

### 3. Ligand-dependent interaction with endothelial cells

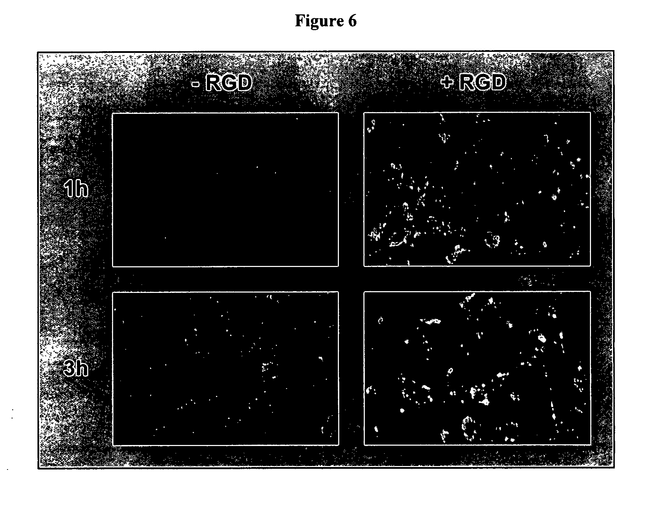

HUVECs were exposed to AVPs for 1 h with or without the RGD ligand that was labeled with a fluorescent lipid (N-(7-nitrobenz-2-oxa-1,3-diazol-4-yl)-1,2-dihexadecanoyl-sn-glycero-3-phospho-ethanolamine, NBD-PE). Cells were observed directly 1 h after incubation with the labeled AVPs (Fig. 6; 1 h) or after another 2 h in normal culture medium (Fig. 6; 3 h) under a fluorescence microscope. At both time points a considerably stronger staining was detectable in cultures that had received the RGD-AVPs. More than 99% of these cells were stained. Confocal microscopy also showed that the staining was not confined to the cell surface, but also occurred intracellularly, indicating endosomal uptake of the RGD-AVPs (data not shown). These observations demonstrate a highly efficient and selective interaction of the RGD-AVPs with endothelial cells (ECs).

### 4. Highly efficient transduction of endothelial cells

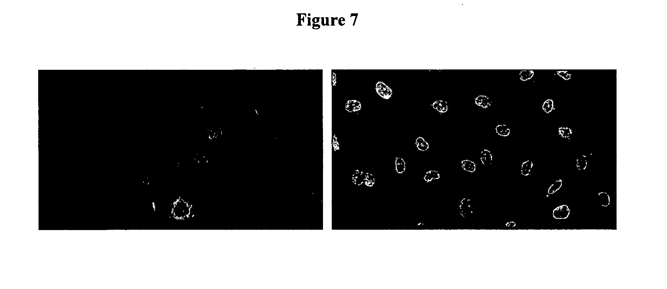

The transduction efficiency of the RGD-AVPs with respect to (i) transduction efficiency, (ii) contribution of the ligand to EC transduction and (iii) cell type specificity was evaluated. For this purpose a plasmid carrying a CMV promoter driven nuclear green fluorescent protein (H2BGFP) gene (Kanda, T., et al. (1998) *Curr. Biol.* **8:** 377-85) was packaged into RGD-AVPs. Cells were exposed for 1 h to the RGD-AVPs, and the fraction of green fluorescent cells was determined 23 h later (Fig. 7). A quantitative evaluation of the experiment showed that the majority of cells were successfully transduced by RGD-AVPs and expressed GFP. These experiments were performed in complete cell culture medium containing 10% FCS, indicating that the RGD-AVPs were not inhibited by serum proteins. When protamine sulfate was used as the DNA-compacting agent instead of PEI, a >50-fold decrease in transduction efficiency was observed. Likewise, the PEI-DNA complex on its own (without lipid envelope) gave poor transduction efficiencies (<5% transduced cells).

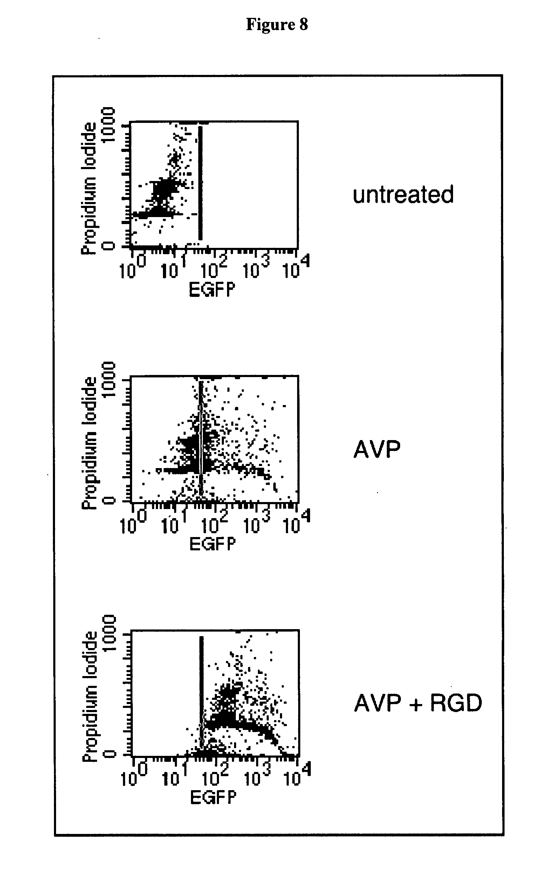

To quantitate the results FACS-analyses of RGD-AVPs was performed and for comparison AVPs lacking the RGD ligand were included. The data in Fig. 8 clearly confirms the dramatic transduction efficiency already seen in the experiment shown in Fig. 7. Approximately 99% of the cells receiving RGD-AVPs showed GFP expression. In contrast, GFP expression was seen in only ∼40% of cells transduced with AVPs lacking the ligand, and the level of this expression was considerably lower than that seen with the ligand-carrying AVPs.

### 5. Cell type selectivity of transduction

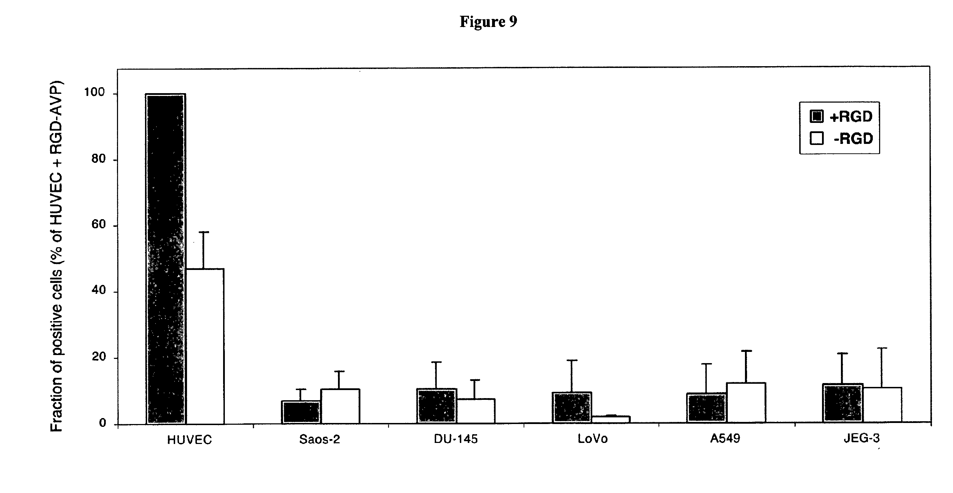

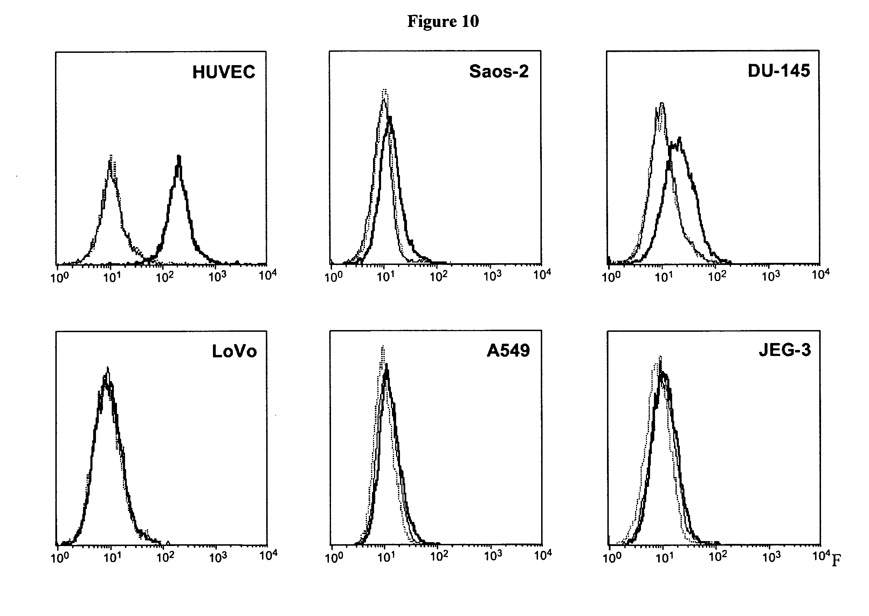

The transduction of HUVECs relative to other cell types which lack αᵥβ₃ integrin expression as determined by immunostaining analysis using antibodies recognizing the heterodimeric αᵥβ₃ integrin complex was analyzed. The results of this experiment are summarized in Figs. 9 and 10. The data obtained with the RGD-AVPs clearly shows that the fraction of GFP-positive cells was approximately 10-fold higher with HUVECs compared to cells expressing no or low levels of αᵥβ₃ integrin, including osteosarcoma cells (Saos-2), prostate carcinoma cells (DU-145), colon carcinoma cells (LoVo), lung adenocarcinoma cells (A549) and choriocarcinoma cells (JEG-3).

Taken together our data indicate that the RGD-AVPs are non-toxic, serum-resistant, exhibit an unprecedented transduction efficiency for ECs and are selective for ECs.

### 6. AVEs

Throughout the preparation of AVEs, synthetic phospholipids were used without further purification. 1,2-dipalmitoleoyl-sn-glycero-3-phosphoethanolamine (DPPE) and 1,2-dioleoyl-sn-glycero-3-[phospho-L-serine] (DOPS) were purchased from Avanti Polar Lipids Inc. (Alabaster, AL), 1,2-dilauroyl-sn-glycero-3-phosphoethanolamine (DLPE) was obtained from Genzyme (Liestal, Switzerland), and cholesterol was from Calbiochem (San Diego, CA). All other substances were of analytical grade. Egg phosphatidylcholine (ePC) was produced by Lipoid KG (Ludwigshafen, Germany) and the anchor lipid 1,2-distearoyl-sn-glycero-3-phosphatidylethanolamine-N-[methoxy(polyethylene glycol)-3400-N-hydroxysuccinimidyl carbonate] (DSPE-PEG-NHS (3400)) was from Shearwater (Huntsville, USA).

### 7. Synthesis of lipid anchor

N-Glutaryl-DPPE was prepared by dissolving DPPE in anhydrous chloroform. Glutaric anhydride and water-free pyridine was added and the solution stirred at 20°C for two days. After this period, the products were dried by applying vacuum to the solution and N-Glutaryl-DPPE was purified using preparative silica gel chromatography (Merck 60 F 254) in chloroform/methanol/ammonia (65/35/3). Spots were dissolved in methanol, dried and redissolved in chloroform for further use. N-Glutaryl-DPPE was identified by ¹H-NMR as described previously (Ahl, P.L., *et al.* (1997) *Biochimica Et Biophysica Acta* **1329**: 370-82). AVEs with the composition DOPS/DLPE/Cholesterol/N-Glutaryl-DPPE (3:3:3:1 mol/mol) were prepared as follows: a chloroform solution of the lipid mixture (total amount 10 µmol) was dried into a thin film in the inner surface of a rotating 100 ml glass vessel warmed by a water bath to 30°C. Residual chloroform was removed by vacuum desiccation for 15 min. The lipid film was hydrated either in 1 ml of sterile 10 mM Tris buffer (pH 7.4) or in 1 ml sterile PBS. After two hours the resulting multilamellar liposome suspension was placed in a 3 ml glass vessel cooled by an ice/water mixture and sonicated for 15 s in a MSE-Soniprep 150 (Zivy AG, Oberwil, Switzerland) equipped with a titanium tip. Sonication was repeated 10 times, each sonication period was followed by a 30 s pause allowing the suspension to cool. The resulting liposome suspension was extruded through polycarbonate membrane filters with a pore size of 50 nm using a standard device (Liposofast) (MacDonald, R.C., *et al.* (1991) *Biochim. Biophys. Acta* **1061:** 297-303) purchased from Avestin, Ottawa, Canada. The liposomes were used up two months after preparation, during which time no significant increase in liposome size could be detected.

### 8. Targeting motif

Using solid phase synthesis (Applied Biosystems Peptide Synthesizer), a cyclic peptide with the amino acid sequence CDCRGDCFC having an additional arginine at the N-terminus was synthesized. Cyclic condensation of the peptide was accomplished by stirring an aqueous solution of the synthesized peptide under access of ambient air. Completion of cyclic condensation was verified by HPLC. After HPLC purification, the peptide was lyophilized and stored at 4°C.

### 9. Covalent attachment of RGD-peptide to liposomal surface (RGD-AVE)

Activation of the N-Glutaryl-DPPE carboxyl group at the liposomal surface (Weissig, V., et al. (1986) *Febs Letters* **202:** 86-90) was achieved by adding 3.5 mg 1-ethyl-3-(3-dimethylaminopropyl) carbodiimide to 400 µL AVE and shaking the suspension for 5 hr in the dark. The resulting active O-acyl-intermediate reacted with added RGD-peptide (250 µg in 150 µL buffer) overnight yielding a covalent coupling of the peptide to the liposomal surface. The RGD-AVE were separated from unbound peptide by Sephadex G25 gel permeation chromatography in Tris buffer 10 mM pH 7.4. Coupling efficiency was monitored using a fluorescently labeled derivative of the RGD-peptide. For this purpose, RGD was conjugated with 5-(4,6-dichlorotriazinyl)aminofluorescein (5-DTAF) (Blakeslee, D. & Baines, M.G. (1976) *J. Immunol. Meth.* **13:** 305) purchased from Molecular Probes (Eugene, OR). 5-DTAF was dissolved in borate buffer (pH 9) and RGD-peptide was added in a 1:4 molar ratio (RGD / 5-DTAF). The solution was stirred overnight and uncoupled 5-DTAF was separated from the labeled peptide by size exclusion chromatography using Sephadex G25. The purified fluorescently labeled RGD was covalently linked to activated N-Glutaryl-DPPE in AVEs as described above. Fluorescence of the labeled AVE was read using a spectrofluorimeter (model LS50B, Perkin Elmer, Überlingen, Germany) and compared to an appropriately diluted 5-DTAF-RGD solution.jkjn

### 10. Condensation of plasmid DNA

Low molecular weight branched polyethylenimine (PEI; Lupasol G100, BASF AG, Ludwigshafen, Germany) was added to 15 µg of plasmid DNA up to a ratio of polyethylenimine-nitrogen:DNA-phosphate of 20.7. Condensation of plasmid DNA was monitored by dye exclusion technique using Picogreen™ (Molecular Probes, Eugene, OR) as described previously (Fahr, A., et al. (1998) *J. Liposome Res.* **8**: 9-12). In another set of experiments, protamine sulfate (USP-quality; EliLilly & Co., Indianapolis, IN) was used as condensing agent.

### 11. Preparation of RGD-AVPs

An RGD-AVE suspension containing 60 µg of lipid was added to 15 µg plasmid DNA pre-complexed with PEI as described above and gently vortexed. The resulting AVPs are ready for transfection experiments. The indicated amounts are sufficient for one 6 cm cell culture dish.

### 12. DNA condensation measurement by dye exclusion

The experimental protocol was followed as described previously (Fahr, A., et al. (1998) *J. Liposome Res.* **8:** 9-12). Each well of a 96-well plate was filled with 95 µl of Tris 10 mM, pH 7.5. Plasmid DNA was added at concentrations of 300 to 1333 ng/ml in each well and the Picogreen™ concentration was adjusted to a ratio of 1 molecule per 3.75 base pairs of DNA. Fluorescence spectrophotometry was performed at an excitation wavelength of 492.5 nm and an emission wavelength of 518 nm. Appropriate amounts of PEI were added to the wells to achieve the desired N/P-ratios.

### 13. Size and zeta potential analysis of vector particles

Dynamic laser light scattering using a commercially available system Zetasizer 4 (Malvern Instruments, Herrenberg, Germany) with PCS-Software Version 1.26III, Malvern) was used. Autocorrelation data were analyzed by a specialized version of CONTIN (Ruf, H., et al. (1989) *Meth. Enzymol.* **172**: 364-390). Zeta potential measurements were performed on a PALS Zeta Potential Analyzer Ver. 3.12 (Brookhaven Instruments, Holtsville, NY).

### 14. Cryogenic transmission electron microscopy

A suspension of AVPs was applied to a holey carbon-foil grid and vitrified by flash-freezing in liquid ethane. The grids were cryo-transferred to the liquid nitrogen-cooled cryoelectron microscope (Philips CM200 FEG, FEI GmbH, Germany). Images were taken at a magnification of 60 000 under liquid nitrogen conditions at 1.5 microns defocus at 160 keV.

### 15. Cell culture

Human umbilical vein endothelial cells (HUVECs) were prepared by the method of Jaffe (in *Biology of Endothelial Cells* (eds. Jaffe, E.) 1-13 (Martinus Nijhoff, Boston, 1986) as modified by Thornton et al. ((1983) *Science* **222**: 623-625), and cultivated in EGM-2 medium (BioWhittaker Europe, Verviers, Belgium). The colon carcinoma cell line LoVo (provided by I. Hart, London, UK), the lung adenocarcinoma cell line A549 (obtained from K. Havemann, Marburg), the prostate carcinoma cell line DU-145 (obtained from G. Aumüller, Marburg), the choriocarcinoma cell line JEG-3 (obtained from A. Wellstein, Georgetown University) and the osteosarcoma cell line Saos-2 (obtained form N. La Thangue, Glasgow) were cultured in DMEM supplemented with 10% fetal calf serum (FCS; BioWhittaker Europe, Verviers, Belgium) and 2 mM L-glutamine. Cells were passaged (1:5 for HUVECs and 1:10 for tumor cell lines) by trypsinisation (0.05% trypsin, 0.02% EDTA) and grown at 37°C in 5% CO₂.

### 16. WST-assay

One day before transfection cells were seeded in 6-well plates (800,000 cells/well). Cells were incubated for 6 h with the transfection reagents. Lipofectamine and Lipofectin were purchased from Gibco-BRL (Eggenstein, Germany). Effectene and Superfect were obtained from Quiagen (Hilden, Germany). The concentrations of the transfection reagents were optimized for transduction efficiencies and low toxicities (see legend to Fig. 4 and 5 for details). The medium was replaced with normal culture medium, and WST-assays were performed either directly or after another 18 h. The supernatant was withdrawn, 500 µl of WST diluted 1:10 in medium were added to each well and incubated for 30 min at 37°C. Aliquots of 100 µL were transferred to 96-well plates, and the plates were read at 440 nm. All measurements were performed as triplicates, and each experiment was repeated at least three times.

### 17. FACS-analysis of GFP expression

Cells were grown on 10 cm dishes to ∼70% confluency and transfected for 1 h with AVPs made up of 45 µg of DNA and 180 µg of AVE. The cells were trypsinized 23 h later, washed once with PBS, fixed in ice-cold 75% ethanol overnight at 4°C, resuspended in PBS, treated with RNase A (Roche; 400 µg/ml) overnight at 4°C and stained with propidium iodide (20 µg/ml) for at least 10 min. The cells were analyzed by flow cytometry (FACS Calibur; Becton Dickinson, Heidelberg, Germany) using a laser excitation at 488 nm.

### 18. FACS-analysis of αᵥβ₃ expression

Cells from a 10 cm dish were detached with 0.2% EDTA (Roche, Mannheim, Germany) washed once with PBS and incubated with the monoclonal anti-αᵥβ₃-antibody LM609 (Chemicon Int., Hofheim, Germany) diluted 1/100 in PBS with 1% FCS. After washing once with PBS, cells were stained with a Cy3-labelled secondary antibody (goat-anti-mouse-Cy3, F(ab)'₂-fragment, Dianova, Hamburg, Germany) diluted 1/200 in PBS with 1% FCS. All incubations were performed on ice for 30 min. PBS washed cells were analyzed with a FACSCalibur (Becton Dickinson, Heidelberg, Germany) using an excitation at 550 nm. The fluorescence was amplified linearly. A minimum of 10,000 cells were analyzed. Unlabelled cells and cells stained with the secondary antibody only were measured as control.

### 19. Construction of AVEs loaded with Gadolinium as diagnostic agent

A series of liposome formulations was prepared using the rotary evaporation technique. Lipids of the appropriate composition were dissolved in an organic solvent (chloroform or chloroform/methanol) and this solution was dried in a rotary evaporator at 35 mbar and 34°C, thus, yielding a thin lipid film. This thin lipid film was further dried in a vacuum chamber at 10 mbar. The lipid composition of the AVE produced comprised the phospholipids phosphatidylserine (DOPS), phosphatidylethanolamine (DLPE), cholesterol, phosphatidylcholine (ePC), 1,2-distearoyl-sn-glycero-3-phosphatidylethanolamine-N-[methoxy (polyethylene glycol)-3400-N-hydroxysuccinimidyl carbonate (DSPE-PEG-NHS) and/or phosphoethanolamine-N-(Glutaryl) (N-Glut-DPPE) in the following ratios:

**Table 1**

| **Type of liposome** | **DOPS** | **DLPE** | **Cho- lesterol** | **ePC** | **N-Glut- DPPE** | **DSPE- PEG-NHS** | **total lipid amount** |
|---|---|---|---|---|---|---|---|
| AVE-3 | 15,00 | 15,00 | 15,0 | 0,00 | 5,00 | 0,00 | 50,00 |
| AVE-3RGD | 15,00 | 15,00 | 15,0 | 0,00 | 5,00 | 0,00 | 50,00 |
| AVE3-PEG-NHS | 15,83 | 15,83, | 15,83 | 0,00 | 0,00 | 2,50 | 50,00 |
| EPC | 0,00 | 0,00 | 0 | 50,00 | 0,00 | 0,00 | 50,00 |

Afterwards the dried film was hydrated in a rotary evaporator with a few glass beads with an aqueous solution buffered to a pH of 5 to 9.5 in order to form multilamellar vesicles. The buffer contained between 0.1 and 1 molar of gadoteridol (Prohance® , Bracco-Byk Gulden, Konstanz, Germany (RS-[10-Hydroxypropyl)-1,4,7,10-tetraazacyclodecane-1,4,7-triacetato(3-)]gadolinium) or Gadobutrol (Schering AG, Berlin, Germany). The total lipid concentration in this system was 20 mg/ml.

The vesicles were subjected to extrusion through polycarbonate membranes having pores with a size of 50 nm. The extrusion was repeated 21 times. Liposomes with a mean size of 90 - 120 nm were obtained, as checked by photon correlation spectroscopy.

Unentrapped metal chelate was removed by gel filtration (Sephadex G25 medium) with phosphate buffer pH 7.4 as elution buffer. Removal of unentrapped metal chelate was also possible using a dialysis method (Mini Lipoprep, Dianorm GmbH, Germany) with a dialysis membrane having a MW cutoff of about 10,000 D. AVEs containing 1 M Gadobutrol where dialyzed with four changes of buffer at 2, 4, 6, 8 h against PBS. The thus obtained liposomes had a diameter of between 100 to 180 nm as measured by a Zetasizer 3000 HS (Malvern Instruments, UK).

A cyclic CDCRGDCFC-Peptide (Pierschbacher, M.D. and Ruoslahti, E. (1984) *Nature* **309:** 30-33; Koivunen, E., et al. (1995) *Biotechnology* **13:** 265-270) with an additional arginine at the N-terminus was synthesized. This terminal amino acid lowers the probability of involving the peptide active center in the subsequent coupling procedure between the peptide and the liposomes.

The coupling for AVEs containing the lipid composition indicated for AVE-3, AVE-3RGD and EPC was done according to Weissig, V., et al. ((1986) *FEBS Lett.* **202:** 86-90; Ezpeleta, I., et al. (1995) *Int. J*. *Pharm.* **142:** 227-233. In short: the carboxyl group of the phosphoethanolamine-N-(Glutaryl) was activated by addition of 1-ethyl-3-(3-dimethylaminopropyl)-carbodiimide (EDC) to the liposomal suspension (molar ratio lipid: EDC = 1: 4.56). This solution was agitated for five hours in the dark. During this time the O-acyl-intermediate of the phosphoethanolamine-N-(Glutaryl) was formed, which is able to react with the RGD peptide. Then an appropriate amount of a RGD-solution was added to the liposome suspension to give a phosphoethanolamine-N-(Glutaryl) : RGD-peptide ratio of about 2.1. This mixture was agitated and stored overnight.

For liposomes containing PEG (AVE-3 PEG-NHS) the coupling was done with 0.3 µmol RGD-peptide dissolved in 125 µl PBS per 1.5 µmol DSPE-PEG-NHS. The resulting dispersion was shaken for two hours at 4°C.

Unbound peptide was separated from the liposome suspension by gel filtration (Sephadex G25 medium) with Tris buffer, pH 7.4.

The resulting liposome suspensions were ready for injection or transfection.

### 20. Transfection of Gd containing liposomes into cells carrying the αᵥβ₃ receptor

Cells carrying the RGD-receptor (HUVEC, MEWO) and cells without the RGD-receptor (A549) as control were used in the following experiments. The cells were grown in 6 well tissue culture dishes under standard conditions As cell culture medium DMEM was used for MEWO and A549 cells. HUVECs were cultured in EGM-2 medium . The growth medium was removed and 500 µl transfection media 199 was applied to each well of the plate and 10-40 µl liposomes (EPC, AVE-3, AVE-3, which were produced using 1 N-Gadobutol, or free Gadobutol as control) were applied to each well. After 1 hour incubation the transfection media was removed and the cells were washed with 2 ml PBS for each well and subsequently the respective growth media required by the cells were added. After 18 h the media was again removed and the cells were washed twice with 2 ml PBS and subsequently 250 µl luciferase lysis buffer (1ml Triton; 25 ml Glycylglycine 0,1 M pH 7,8; 1,5 ml MgSO₄ 1M; 1,6 ml EGTA 0,25 M and 71,9 ml purified water) was added. The lysates were transferred into 1.5 ml tubes and the T1-relaxation times were measured in a magnetic resonance tomograph for each probe.

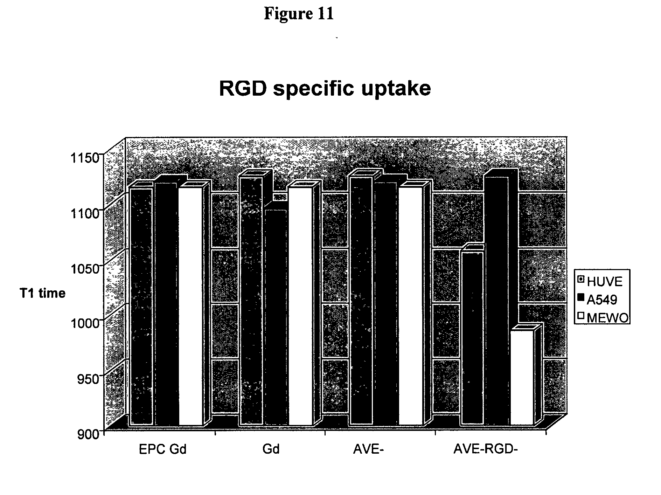

As can be seen from figure 11, the T1 relaxation time was shortened in samples obtained from AVE-RGD-Gd treated cells and only in cells carrying the RGD-receptor. The lysates of A549 cells did not show a significant increase of the T-1 relaxation time.

### 21. Tumor imaging with AVE-PEG-RGD liposomes

The following experiments show the results of *in vivo* tumor imaging using AVE-RGD-Gd and AVE-PEG-RGD-Gd liposomes. NUDE-mice were inoculated with MEWO tumor cells in the back which was allowed to form a tumor for 14 days. Subsequently, AVE-RGD-Gd liposomes or AVE-PEG-RGD-Gd liposomes were injected into the tail vein of male anastesized NUDE-mice. The total amount of injected Gadolinium was 0.1 µmol/g mouse. The mouse body was analyzed by MRT immediately after injection and at different time points (after 10, 30, 45, 60 and 75 minutes)

As can be seen from Figure 12 panel AVERGD-Gd liposomes were enriched after a short time of 5 minutes in the liver and in spleen of the mice and there was a marked increase in tumor tissue observed for the AVE-PEG-RGD-Gd liposomes. This signal was particularly prominent one hour after injection. In AVE-PEG-RGD Gd liposome treated mice neither liver nor spleen showed an increase in signal. However, for both liposome formulations an increase in the signal was detectable in kidney and bladder.

Priority application US 60/252,666 filed November 22, 2000 including the specification, drawings, claims, sequence listing and abstract, is hereby incorporated by reference. All publication cited herein, are incorporated in their entireties by reference.

## Claims

1. A diagnostic system comprising:
(a) at least one negatively charged carrier;
(b) at least one targeting moiety; and
(c) at least one diagnostic agent.

2. The diagnostic system according to claim 1, wherein said negatively charged carrier is an anionic lipid.

3. The diagnostic system according to claim 2, wherein said anionic lipid is selected from the group consisting of phosphatidylserine, phosphatidylglycerol, phosphatidylacid, phosphatidylinositol, and cholesterolglutarate.

4. The diagnostic system according to one of claims 1-3, wherein said targeting moiety is selected from the group consisting of a peptide, a protein, an antibody, an antibody fragment, a single-chain antibody, a diabody or a small molecule.

5. The diagnostic system according to one of claims 1-4, wherein said diagnostic agent is selected from the group consisting of an electron dense substance, a paramagnetic substance, a superparamagnetic substance, a radioactive substance, a fluorescent substance, a luminescent substance, a light-emitting substance, a genetic construct that codes for a fluorescent protein and a genetic construct that codes for a protein detectable by staining procedures.

6. The diagnostic system according to one of claims 1-5, further comprising at least one uncharged carrier.

7. The diagnostic system according to claim 6, wherein said uncharged carrier is selected from the group consisting of cholesterol, phosphatidylcholine, and phosphatidylethanolamine.

8. The diagnostic system according to one of claims 1-7, further comprising at least one amphiphatic polymer.

9. The diagnostic system according to claim 8, wherein the amphiphatic polymer is selected from the group of polyethylene glycol (PEG), poly(2-methyl-2-oxazoline) (PMOZ), and poly(2-ethyl-2-oxazoline) PEOZ.

10. The diagnostic system according to one of claims 8-9, wherein the amphiphatic polymer is attached to a lipid,

11. The diagnostic system according to claim 10, wherein the lipid is selected from alpha-methoxy-omega-(1,2-dioctadecenoyloxy glyceryl) (DO), alpha-methoxy-omega-(1,2-ditetradecenoyloxy glyceryl) (DT), distearoylphosphatidyl (DSPE), and alpha-(dipalmitoylphosphatidyl) (DPP).

12. The diagnostic system according to one of claims 8-11, wherein the amphiphatic polymer has a molecular weight between 1.000 and 5.000 Da.

13. The diagnostic system to one of claims 1-12, wherein the negative carrier of the diagnostic system forms at least part of a lamellar structure.

14. The diagnostic system of claim 13, wherein the lamellar structure is a unilamellar, bilamellar, oligolamellar or polylamellar structure.

15. The diagnostic system to one of claims 13-14, wherein said lamellar structure is a liposome.

16. The diagnostic system according to one of claims 13-15, wherein said lamellar structure is an artificial viral envelope.

17. The diagnostic system according to one of claims 13-16, wherein said lamellar structure comprises a payload compartment.

18. The diagnostic system according to claim 17, wherein the diagnostic agent is encapsulated within said payload compartment.

19. The diagnostic system to one of claims 6-18, wherein at least one targeting moiety is covalently attached to at least one of said negatively charged carriers or uncharged carriers.

20. A method for producing the diagnostic system according to one of claims 1-19, wherein at least one lamella is formed with at least one negatively charged carrier and at least one diagnostic agent in solution.

21. The method according to claim 20, wherein at least one targeting moiety is covalently attached after formation of at least one lamella.

22. The method according to claim 20, wherein at least one targeting moiety is mixed with said at least one negatively charged carrier.

23. A method for producing the diagnostic system according to one of claims 1-19, wherein at least one lamella is loaded with at least one diagnostic agent upon formation of the lamella.

24. Use of the diagnostic system according to one of claims 1-19 for the manufacturing of a medicament for the diagnosis of a disease.

25. The use according to claim 24, wherein said disease is a tumor disease.

26. A method of using the diagnostic system according to one of claims 1-19 for diagnosis of cells and tissues *in vitro.*

27. A method of using the diagnostic system according to one of claims 1-19 for the diagnosis of microbiological organisms *in vitro.*

28. A diagnostic kit comprising:
(a) at least one negatively charged carrier; and
(b) at least one diagnostic agent.

29. The diagnostic kit according to claim 28 further comprising at least one targeting moiety.
